# FASCICULE DE BREVET EUROPEEN

(11) **EP 0 794 949 B1**
(45) Date de publication et mention de la délivrance du brevet: **29.07.1998**
(21) Numéro de dépôt: 95941754.4
(22) Date de dépôt: 01.12.1995
(51) Int. Cl.: C07D 253/06, C07D 405/12, A61K 31/53

(54) **NOUVEAUX DERIVES DE 3,5-DIOXO-(2H,4H)-1,2,4-TRIAZINES, LEUR PREPARATION ET LEUR APPLICATION A TITRE DE MEDICAMENT**
3,5-DIOXO-(2H,4H)-1,2,4-TRIAZINDERIVATE IHRE HERSTELLUNG UND VERWENDUNG ALS MEDIKAMENT
NOVEL 3,5-DIOXO-(2H,4H)-1,2,4-TRIAZINE DERIVATIVES, THEIR PREPARATION AND USE AS DRUGS

(30) Priorité: 02.12.1994 FR 9414544
(43) Date de publication de la demande: 17.09.1997
(73) Titulaire: PIERRE FABRE MEDICAMENT, 92100 Boulogne (FR)
(72) Inventeur: PATOISEAU, Jean-François, F-81100 Castres (FR); FAURE, Christian, F-31200 Toulouse (FR); DUPONT-PASSELAIGUE, Elisabeth, F-81100 Castres (FR); COURET, Françoise, F-31450 Corransac (FR); KOEK, Wouter, F-81290 Viviers-les-Montagnes (FR)
(74) Mandataire: Ahner, Francis
(86) Numéro de dépôt international: FR9501589
(87) Numéro de publication internationale: WO9616949

(56) Documents cités:
- EP-A- 0 512 755
- EP-A- 0 527 081
- EP-A- 0 559 285
- WO-A-95/01965
- US-A- 5 036 070

## Description

La présente invention a pour objet de nouveaux dérivés de la 3,5-dioxo-(2H, 4H)-1,2,4-triazine fonctionnalisés en 2, leur préparation et leur application en thérapeutique.

Dans le cadre de la recherche de nouveaux médicaments anxiolytiques à profil non benzodiazépiniques, la découverte et le développement de la buspirone ont suscité un nombre important de travaux. De multiples observations permettent d'associer un dysfonctionnement du système sérotoninergique à certaines pathologies psychiatriques telles que l'anxiété ou la dépression (M. Hamon, H. Gozlan, Médecine/Sciences 1993, 9, 21-30). Ainsi, ces dernières années de nombreux composés présentant une affinité vis-à-vis des récepteurs 5HT_{1A} ont été revendiqués pour leur intérêt en thérapeutique humaine et plus particulièrement pour leur activité anxiolytique (J. Peergaard et al., current opinion in therapeutic patents, Janvier 1993, 101-128).

Des dérivés de la 3,5-dioxo-6-amino-(2H, 4H)-1,2,4-triazine ont été revendiqués par la demanderesse pour leur application en thérapeutique humaine (FR dépôt N° 93.08259/06.07.93).

Les composés de la présente invention se caractérisent par leur structure originale, leur puissante affinité vis-à-vis du récepteur 5HT_{1A} et leur profil pharmacologique.

Les composés de l'invention correspondent à la formule I, 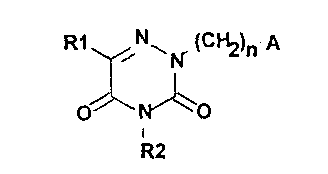 dans laquelle
- R₁ représente l'hydrogène, un radical alcoyle en C₁-C₄, phényl alcoyle en C₁-C₄ ou phényle, le noyau phényle étant éventuellement substitué par un ou plusieurs groupements tels que alcoyle en C₁-C₃, hydroxyle, trifluorométhyle ou halogène,
- R₂ représente l'hydrogène ou un radical alcoyle en C₁-C₄,
- n peut prendre les valeurs entières de 2 à 6,
- A représente un groupement de type
   . aryl piperazino II 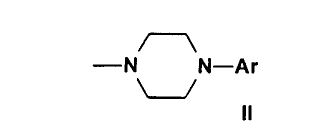 le groupement Ar représentant lui-même une structure aromatique telle que phényle, naphtyle, pyrimidyle, pyridyle, éventuellement substituée par un ou plusieurs groupements tels que alcoyle en C₁-C₃, alcoxy en C₁-C₃, hydroxy, trifluorométhyle ou halogène,
   . benzodioxanyl méthylamino ou pyridodioxanyl méthyl amino III 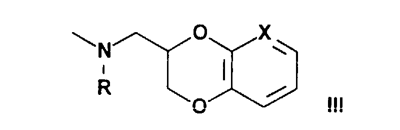 dans lequel R représente l'hydrogène ou un groupement alcoyle en C₁-C₃ et X représente un atome d'azote ou de carbone.

En outre, l'invention couvre les sels de composés de formule générale I avec les acides pharmaceutiquement acceptables, ainsi que les différents énantiomères dans le cas de composés possédant un carbone asymétrique.

En particulier, n peut prendre les valeurs 2, 3 ou 4.

Les composés de l'invention peuvent être obtenus selon deux voies de synthèse différentes.

### . Méthode A.

Elle est caractérisée en ce que :
1 - l'on traite un composé de formule générale IV 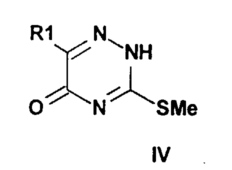 dans laquelle R₁ est tel que défini dans la formule I avec un dérivé dihalogéné de formule V

   Hal-(CH₂)n-Hal' V

   dans laquelle n est tel que défini dans la formule I, et Hal et Hal' représentent un halogène, de préférence respectivement le chlore pour Hal et le brome pour Hal'. La réaction est conduite dans le diméthyl formamide en présence d'hydrure de sodium.
2 - Après hydrolyse acide, on traite par un dérivé de formule VI ou VII 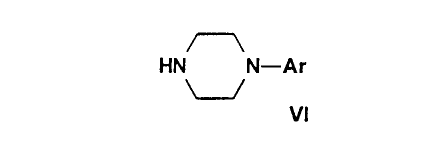 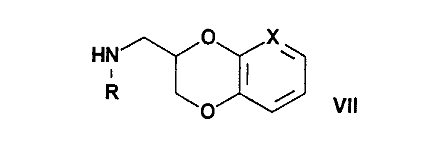 dans lesquels Ar, R et X sont tels que définis dans les formules I, II et III.
   La réaction est effectuée par chauffage dans le toluène ou le xylène ou dans le butanol en présence de triéthylamine.
3 - On traite optionnellement par un dérivé R₂Y dans lequel R₂ est tel que défini dans la formule I et Y représentant le chlore, le brome ou l'iode, dans le diméthyl formamide en présence d'hydrure de sodium.

### . Méthode B.

Elle est caractérisée en ce que :
1 - On traite un composé de formule VIII : 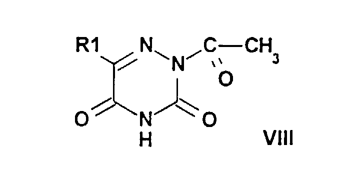 dans lequel R₁ est tel que défini dans la formule I par un halogénure d'alcoyle R₂Y, dans le diméthyl formamide en présence d'hydrure de sodium.
2 - On désacétyle en milieu acide tel que l'acide paratoluène sulfonique dans l'éthanol.
3 - On traite par un composé dihalogéné V tel que défini précédemment, dans le diméthyl formamide en présence d'hydrure de sodium, puis par un dérivé VI ou VII, tels que définis précédemment.

Les composés intermédiaires et finaux peuvent être, si on le désire, purifiés suivant une ou plusieurs méthodes de purification choisies parmi, l'extraction, la filtration, la chromatographie sur gel de silice, la cristallisation.

Les matières premières utilisées dans les procédés décrits ci-dessus sont commerciaux ou aisément accessibles à l'homme de métier selon des procédés décrits dans la littérature.

Les exemples suivants illustrent l'invention sans en limiter la portée.

Les analyses élémentaires et les spectres IR et RMN confirment les structures des composés obtenus selon l'invention.

### Exemple 1 : 2-[4-(4-(3-trifluorométhylphényl)pipérazino)butyl]-3,5-dioxo-(2H, 4H)-1,2,4-triazine chlorhydrate 1 (méthode A).

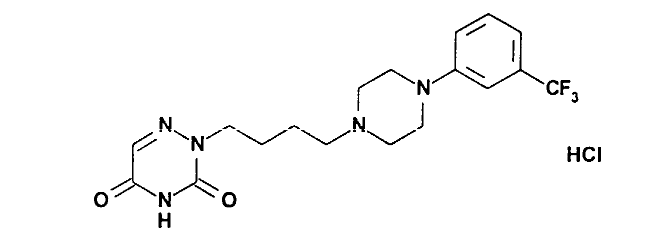

### a) 2-(4-chloro butyl)-3-méthylthio-5-oxo-(2H)-1,2,4-triazine 1a.

A une suspension d'hydrure de sodium à 60 % dans l'huile de paraffine (3,52 g ; 0,088 mole) dans le DMF (40 ml), est ajoutée goutte à goutte une solution de 3-méthylthio-5-oxo-(2H)-1,2,4 triazine (11,45 g ; 0,08 mole) dans le DMF (100 ml). Après une heure d'agitation à température ambiante, on ajoute le 1-bromo-4-méthylthio-5-oxo-(2H)-1,2,4 triazine (11,45 g ; 0,08 mole) dans le DMF (100 ml).

Après une heure d'agitation à température ambiante, on ajoute le 1-bromo-4 chlorobutane (15 g ; 0,088 mole) et maintient une nuit sous agitation. Après concentration à sec sous vide le résidu est repris par l'eau (20 ml) et extrait au chlorure de méthylène (2 x 50 ml). Les phases organiques sont séchées (Na₂SO₄) et concentrées à sec sous vide pour donner le composé 1a sous forme d'une huile brune (12,4 g).
CCM : gel de silice 60F254 Merck
Toluène - dioxane - triéthylamine 80 - 15 - 5
Rf= 0,33.

### b) 2-(4-chlorobutyl)-3,5-dioxo-(2H, 4H)-1,2,4-triazine 1b.

Le composé 1a (16 g) est chauffé dans l'acide chlorhydrique 2N (80 ml) à 100°C pendant 30 minutes. Après refroidissement, le mélange est extrait au chlorure de méthylène (2 x 50 ml). Les phases organiques sont séchées (Na₂SO₄), évaporées à sec sous vide puis reprises par l'éther éthylique bouillant (100 ml). Après concentration sous vide et empatage au toluène, on obtient par filtration et séchage à 60°C sous vide, le composé 1b (2,62 g).
F = 93°C
CCM : gel de silice 60F254 Merck
CHCl₃ - MeOH 95 - 5 ; Rf= 0,35.

### c) 2-(4-(4-(3-trifluorométhylphényl)pipérazino)butyl)-3,5-dioxo-(2H,4H)-1,2,4-triazine chlorhydrate 1.

Le composé 1b (6,67 g ; 0,033 mole) et la 4-(3-trifluorométhylphényl) pipérazine (15,3 g ; 0,066 mole) sont chauffés à sec pendant 7 heures à 120 - 130°C. On ajoute du xylène (40 ml) et on chauffe une heure supplémentaire à reflux. Après refroidissement et concentration à sec sous vide, le résidu est repris dans l'eau (30 ml) et dans l'éther éthylique (2 x 100 ml). Les phases organiques sont séchées (Na₂SO₄) et concentrées à sec sous vide. L'huile obtenue est reprise par l'éther éthylique (50 ml) puis additionnée goutte à goutte d'acide chlorhydrique dans l'éther éthylique (20 ml). On essore un solide qui est cristallisé dans l'éthanol (50 ml). Après filtration, lavage à l'éther éthylique et séchage sous vide à 80°C, on obtient le composé 1 (3,77 g).
F = 200°C
CCM : gel de silice 60F254 Merck
CHCl₃ - MeOH 95 - 5 ; Rf= 0,27.

### Exemple 2: 4-méthyl-2-(4-(4(3-trifluorométhylphényl)-pipérazino)-butyl)-3,5-dioxo-(2H,4H)-1,2,4-triazine 2.

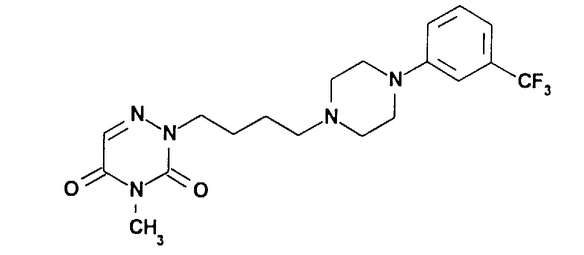

A une suspension d'hydrure de sodium à 60 % dans l'huile de paraffine (0,77 g ; 0,019 mole) dans le DMF (50 ml) on ajoute goutte à goutte le composé 1 (3,77 g ; 0,009 mole) puis après 2 heures d'agitation à température ambiante l'iodométhane (1,4 g ; 0,01 mole). Après une nuit à température ambiante, le mélange est concentré à sec sous vide puis le résidu repris par l'eau (100 ml) et extrait à l'éther éthylique (2 x 50 ml). Les phases organiques séchées (Na₂SO₄) sont concentrées à sec sous vide pour fournir une huile qui cristallise lentement.

Après recristallisation dans le 2-propanol et séchage sous vide à 60°C, on obtient le composé 2 (2 g).
F = 77°C
CCM : gel de silice 60F254 Merck
CHCl₃ - MeOH 95 - 5 ; Rf= 0,52.

### Exemple 3 : 4-méthyl-2-(4-(4-(3-trifluorométhylphényl)pipérazino)-butyl)-3,5-dioxo-(2H, 4H)-1,2,4-triazine chlorhydrate 3.

Le composé 2 (1,9 g) repris dans l'éther éthylique (40 ml) est additionné d'une solution d'acide chlorhydrique dans l'éther éthylique. Le précipité blanc obtenu est, après filtration, recristallisé dans l'éthanol (60 ml) et séché sous vide à 80°C pour donner le composé 3 (1,5 g).
F = 225°C
CCM : gel de silice 60F254 Merck
CHCl₃ - MeOH 95 - 5 ; Rf= 0,52.

### Exemple 4 : 4-méthyl-2-(4-(4-(3-chlorophényl)pipérazino)butyl)-3,5-dioxo-(2H, 4H)-1,2,4-triazine 4 (méthode B).

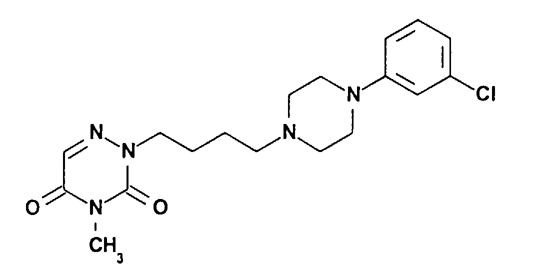

### a) 2-acétyl-3,5-dioxo-(2H, 4H)-1,2,4-triazine 4a.

L'azauracile (50 g) est traité pendant 80 minutes au reflux de l'anhydride acétique (300 ml). Après refroidissement, et concentration à sec sous vide, on obient par empatage au toluène (300 ml) le composé 4a (62,2 g).
F = 148°C
CCM : gel de silice 60F254
CHCl₃ - MeOH 90 - 10 ; Rf= 0,38.

### b) 4-méthyl-3,5-dioxo-(2H, 4H)-1,2,4-triazine 4b.

A une suspension d'hydrure de sodium à 60 % (0,44 g ; 0,011 mole) dans le DMF (25 ml), est ajouté le composé 4a (1,55 g ; 0,01 mole). Après 1 heure sous agitation à température ambiante, on ajoute l'iodométhane (1,56 g ; 0,011 mole) et maintient une nuit sous agitation. Après concentration à sec sous vide, le résidu est repris par l'éthanol et l'acide p. toluène sulfonique (0,2 g) puis chauffé 2 heures au reflux. Après concentration à sec sous vide et reprise par l'eau (5 ml), on extrait au chlorure de méthylène, on sèche les phases organiques (Na₂SO₄) et on concentre à sec sous vide. Après recristallisation dans le toluène (7 ml) et séchage à 50°C, on obtient le composé 4b (0,74g).
F = 173°C
CCM : gel de silice 60F254 Merck
CHCl3 - MeOH 95 - 5 ; Rf= 0,49.

### c) 4-méthyl-2-(4-chlorobutyl)-3,5-dioxo-(2H, 4H)-1,2,4-triazine 4c.

A une suspension d'hydrure de sodium à 60 % (2,46 g ; 0,062 mole) dans le DMF (25 ml) on ajoute le composé 4b (7,11 g ; 0,056 mole) dans le DMF (70 ml). Après agitation 2 heures à température ambiante, on ajoute le 1-bromo-4-chlorobutane (10,55 g ; 0,062 mole) et on laisse une nuit sous agitation. Après concentration à sec sous vide le résidu est repris à l'eau (15 ml) et extrait à l'éther éthylique (2 x 50 ml). Les phases organiques séchées (Na₂SO₄) et concentrées à sec sous vide conduisent au composé huileux 4c.
CCM : gel de silice 60F254 Merck
Toluène - Acétate d'éthyle 70 - 30 ; Rf = 0,47.

### d) 4-méthyl-2-(4-(4-(3-chlorophényl)pipérazino)butyl)-3,5-dioxo-(2H, 4H)-1,2,4-triazine 4.

Le composé 4c (5,5 g ; 0,025 mole) et la 4-(3-chlorophényl)pipérazine (9,83 g ; 0,05 mole) sont chauffés pendant 3 heures au reflux du butanol (150 ml) en présence de triéthylamine (10 ml). Le mélange est concentré à sec sous vide, repris à l'eau puis extrait à l'éther éthylique (2 x 50 ml). Les phases organiques séchées (Na₂SO₄) sont concentrées à sec sous vide pour donner une huile qui cristallise dans l'éther isopropylique (70 ml). Après recristallisation dans le 2-propanol et séchage sous vide à 40°C, on obtient le composé 4 (4,24 g).
F = 60 - 62°C
CCM : gel de silice 60F254 Merck
CHCl₃ - MeOH 95 - 5 ; Rf= 0,41.

### Exemple 5 : 4-méthyl-2-(4-(4-(3-chlorophényl)pipérazino)butyl)-3,5-dioxo-(2H, 4H)-1,2,4-triazine chlorhydrate 5.

Le composé 4 (2,5 g) est repris dans l'éthanol (50 ml) et additionné d'une solution saturée d'acide chlorhydrique dans l'éthanol. Après filtration, le précipité est empâté dans l'éthanol bouillant (50 ml), essoré, lavé à l'éther éthylique et séché sous vide à 80°C pour donner le 5 (2,68 g).
F = 239°C
CCM : gel de silice 60F254 Merck
CHCl₃ - MeOH 95 - 5 ; Rf= 0,43.

### Exemple 6 : 6-phényl-2-(4-(4-(3-trifluorométhylphényl)pipérazino)-butyl)-3,5-dioxo-(2H, 4H)-1,2, 4- triazine chlorhydrate 6.

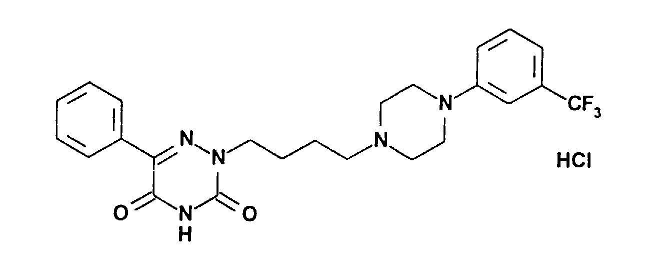

Ce composé est préparé selon le procédé décrit à l'exemple 1, en utilisant au stade a) la 3-méthylthio-5-oxo-6-phenyl-(2H)-1,2,4-triazine.
F = 100°C
CCM : gel de silice 60F254 Merck
CHCl₃ - MeOH 95 - 5 ; Rf= 0,42.

### Exemple 7 : 4-méthyl-6-phényl-2-(4-(4-(3 trifluorométhylphényl)-pipérazino)-butyl-3,5-dioxo-(2H, 4H)-1,2,4-triazine chlorhydrate 7.

Ce composé est préparé à partir du composé 6 selon les procédés décrits aux exemples 2 et 3.
F = 89°C
CCM : gel de silice 60F254 Merck
Toluène - Dioxane - Triéthylamine 80 - 15 - 5 ; Rf = 0,49.

### Exemple 8 : 2-[4-(4-pyrimidin-2- yl pipérazino)butyl]-3-5-dioxo-(2H, 4H)-1,2,4-triazine 8.

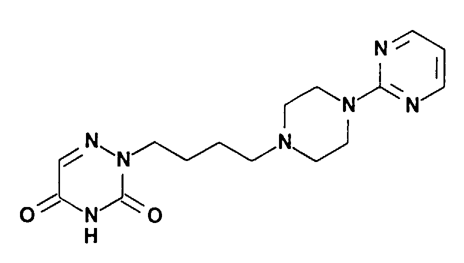

Ce composé est préparé selon le procédé décrit à l'exemple 1, en utilisant au stade c) la 4-pyrimidin-2-yl pipérazine.
F = 139°C
CCM : gel de silice 60F254 Merck
CHCl₃ - MeOH 90 - 10 ; Rf= 0,29.

### Exemple 9 : 4-méthyl-2-(4-(4-pyrimidinyl-2)pipérazino)butyl)-3,5-dioxo-(2H, 4H)-1,2,4-triazine 9.

Ce composé est préparé à partir du composé 8 selon le procédé décrit à l'exemple 2.
F = 93°C
CCM : gel de silice 60F254 Merck
CHCl₃ - MeOH 95 - 5 Rf= 0,40.

### Exemple 10 : 4-méthyl-2-(4-(4-(7-méthoxynaphta1ène-1 yl) pipérazino) butyl)-3,5-dioxo-(2H, 4H)-1,2,4-triazine fumarate 10.

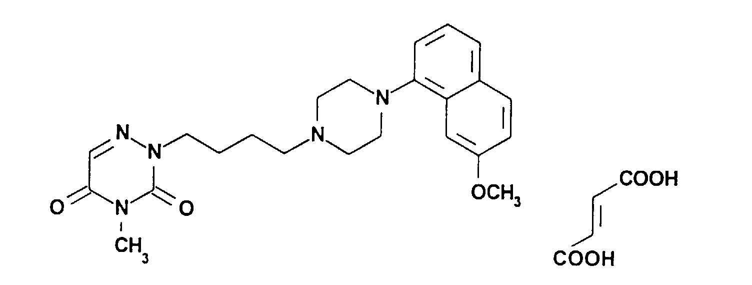

Ce composé est préparé selon le procédé décrit à l'exemple 4 en utilisant au stade d) la 4-(7-méthoxy-1-naphtyl) pipérazine et en salifiant par l'acide fumarique.
F = 172°C
CCM : gel de silice 60F254 Merck
CH₂Cl₂ - MeOH 90 - 10 ; Rf= 0,68.

### Exemple 11 : 4-méthyl-2-(4-(4-(2-méthoxyphényl)pipérazino)butyl)-3,5-dioxo-(2H,4H)-1,2,4-triazine 11.

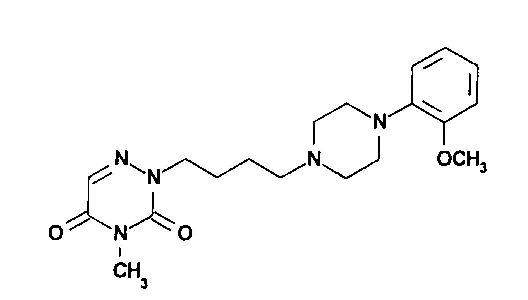

Ce composé est préparé selon le procédé décrit à l'exemple 4 en utilisant au stade a) la 4-(2-méthoxyphényl)pipérazine.
F = 72 - 74°C
CCM : gel de silice 60F254 Merck
CHCl₃ - MeOH 95 - 5 ; Rf = 0,31.

### Exemple 12 : 6-méthyl-2-(4-(4-(3-trifluorométhylphényl)pipérazino)-butyl)-3,5-dioxo-(2H, 4H)-1,2,4-triazine 12.

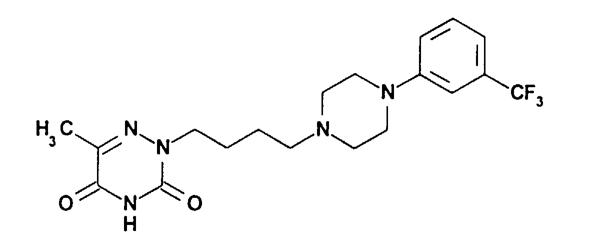

Ce composé est préparé selon le procédé décrit à l'exemple 1, en utilisant au stade a) la 3-méthylthio-6-méthyl-5-oxo-(2H, 4H)-1,2,4-triazine.
F = 123°C
CCM : gel de silice 60F254 Merck
CHCl₃ - MeOH 95 - 5 ; Rf= 0,30.

### Exemple 13 : 4,6-diméthyl-2-(4-(4-(3-trifluorométhylphényl)pipérazino) butyl)-3,5-dioxo-(2H,4H)-1,2,4-triazine chlorhydrate 13.

Ce composé est préparé à partir du dérivé 12 selon les procédés décrits aux exemples 2 et 3.
F = 208°C
CCM : gel de silice 60F254 Merck
CHCl₃ - MeOH 95 - 5 ; Rf= 0,66.

### Exemple 14 : 4-méthyl-2-(4-(1,4-benzodioxane-2-yl méthylamino) butyl)-3,5-dioxo-(2H, 4H)-1,2,4-triazine 14.

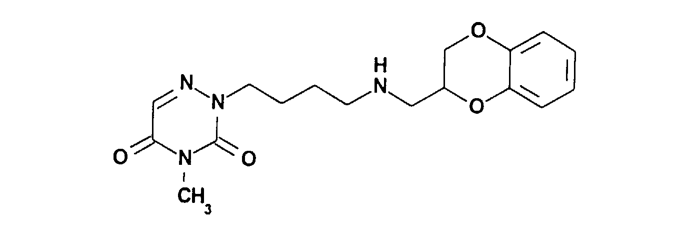

Ce composé est préparé selon le procédé décrit à l'exemple 4 en utilisant au stade d) la 1,4-benzodioxan-2-yl méthylamine.
F = 70 - 72°C
CCM : gel de silice 60F254 Merck
CHCl₃ - MeOH 95 - 5 ; Rf = 0;31.

### Exemple 15 : 4-méthyl-2-(4-(N-méthyl, 1,4-benzodioxan-2-yl méthylamino)butyl)-3,5-dioxo-(2H, 4H)-1,2,4-triazine fumarate 15.

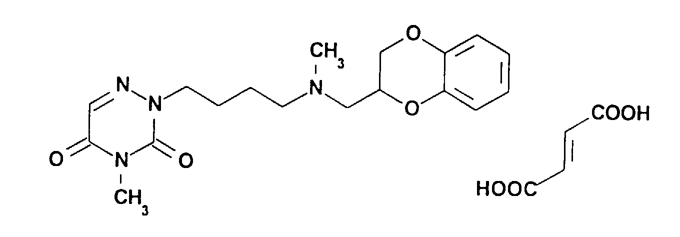

Le composé 14 (1,8 g) dans l'acide formique (48 ml) est traité par le formaldéhyde à 37 % (50 ml) à 100°C pendant 10 heures. Après concentration à sec sous vide, le résidu est repris à l'eau, alcalinisé à pH 11 et extrait à l'acétate d'éthyle (3 x 200 ml). Les phases organiques séchées (Na₂SO₄) sont concentrées à sec sous vide et purifiées par flash chromatographie sur silice (éluant CH₂Cl₂-MeOH 95 - 5). On obtient par salification à l'acide fumarique le composé 15 (0,18 g).
F = 135°C
CCM : gel de silice 60F254 Merck
CH₂Cl₂ - MeOH 95 - 5 ; Rf= 0,36.

### Exemple 16 : 4-méthyl-2-(4-(2,3-dihydro-1,4-dioxino-[2,3-b]-pyridin-2-yl méthylamino)butyl)-3,5-dioxo-(2H, 4H)-1,2,4-triazine fumarate 16.

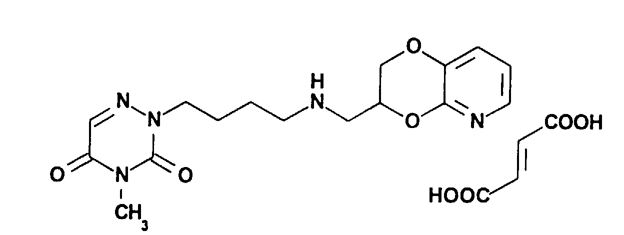

Ce composé est obtenu selon le procédé décrit à l'exemple 4 en utilisant au stade d) le 2,3-dihydro-1,4-dioxino-[2,3-b]-pyridin-2-yl méthylamine.
F = 147 - 148°C
CCM : gel de silice 60F254 Merck
CH₂Cl₂ - MeOH - NH₄OH 90 - 9 - 1 ; Rf= 0,40.

### Exemple 17 : 2-(3-(4-(3-trifluorométhyphényl)pipérazino)propyl)-3,5-dioxo-(2H, 4H)-1,2,4-triazine 17.

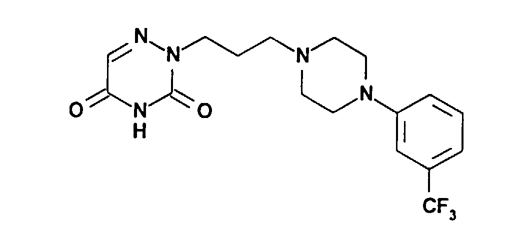

Ce composé est obtenu selon le procédé décrit à l'exemple 1 en utilisant au stade la) le 1-bromo-3-chloropropane.
F = 140°C
CCM : gel de silice 60F254 Merck
CHCl₃ - MeOH 95 - 5 ; Rf = 0,21.

### Exemple 18 : 2-(2-(4-(3-trifluorométhylphényl)pipérazino)éthyl)-3,5-dioxo-(2H, 4H)-1,2,4-triazine chlorhydrate 18.

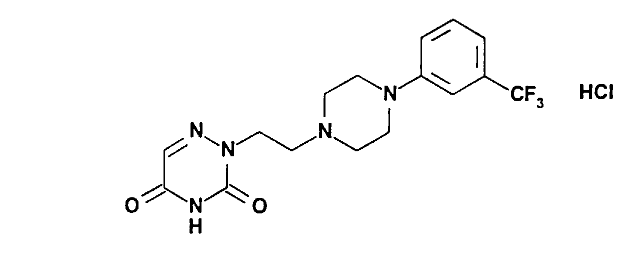

Ce composé est obtenu selon le procédé décrit à l'exemple 1 en utilisant au stade 1a) le 1-bromo-2-chloro éthane.
F = 250°C
CCM : gel de silice 60F254 Merck
CHCl₃ - MeOH 95 - 5 ; Rf= 0,32.

### Exemple 19 : 4-méthyl-2-(3-(1,4-benzodioxan-2-yl méthylamino)-propyl)-3,5-dioxo-(2H, 4H)-1,2,4-triazine fumarate.

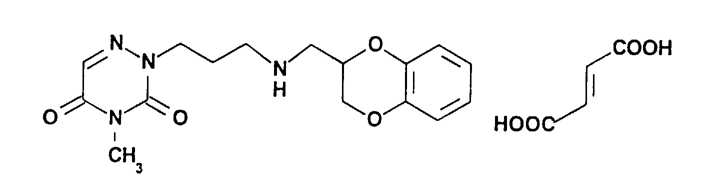

Ce composé est obtenu selon le procédé décrit à l'exemple 4 en utilisant au stade 4c) le 1-bromo-3-chloropropane et au stade 4d) la 1,4-benzodioxan-2-yl méthylamine.
F = 161°C
CCM : gel de silice 60F254 Merck
CH₂Cl₂ - MeOH 90 - 20 ; Rf= 0,63.

### Exemple 20: 4-méthyl-2-(3-(2,3 -dihydro-1,4-dioxino-[2,3-b]-pyridin-2-yl méthylamino)propyl)-3,5-dioxo-(2H, 4H)-1,2,4-triazine fumarate 20.

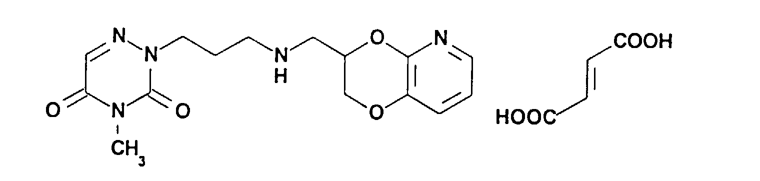

Ce composé est obtenu selon le procédé décrit à l'exemple 4 en utilisant au stade 4c) le 1-bromo-3-chloropropane et au stade 4d) la 2,3-dihydro-1,4-dioxino-[2,3-b]-pyridin-2-yl méthylamine.
F = 156°C
CCM : gel de silice 60F254 Merck
CH₂Cl₂ - MeOH 90 - 10 ; Rf = 0,45.

### Exemple 21 : 4-méthyl-2-(4-(4-(3,4-dichlorophényl)pipérazino)butyl)-3,5-dioxo-(2H, 4H)-1,2,4-triazine 21.

Ce composé est préparé selon le procédé décrit à l'exemple 4 en utilisant au stade d) la 4-(3,4-dichlorophényl)pipérazine.
F = 74°C
CCM : gel de silice 60F254 Merck
CH₂Cl₂ - MeOH 95-5 Rf= 0,45.

### Exemple 22: 4-méthyl-2-(4-(4-(2,3-dichlorophényl)pipérazino)butyl)-3,5-dioxo-(2H, 4H)-1,2,4-triazine 22.

Ce composé est préparé selon le procédé décrit à l'exemple 4 en utilisant au stade d) la 4-(2,3-dichlorophényl)pipérazine.
F = 116°C
CCM : gel de silice 6UF254 Merck
CH₂Cl₂ - MeOH 95-5 Rf= 0,30.

### Exemple 23: 4-méthyl-2-(4-(4-(3,5-dichlorophényl)pipérazino)butyl)-3,5-dioxo-(2H, 4H)-1,2,4-triazine 23.

Ce composé est préparé selon le procédé décrit à l'exemple 4 en utilisant au stade d) la 4-(3,5-dichlorophényl)pipérazine.
F = 128°C
CCM : gel de silice 60F254 Merck
CH₂Cl₂ - MeOH 95-5 Rf= 0,35.

### Exemple 24 : 4-méthyl-2-(4-(4-(4-chloro-2-pyrimidinyl)pipérazino)butyl-3,5-dioxo-(2H, 4H)-1,2,4-triazine 24.

Ce composé est préparé selon le procédé décrit à l'exemple 4 en utilisant au stade d) la 4-(4-chloro-2-pyrimidinyl)pipérazine.
F = 104°C
CCM : gel de silice 60F254 Merck
CH₂Cl₂ - MeOH 90-10 Rf= 0,55.

### Exemple 25 : 4-méthyl-2-(4-(4-(4-methyl-2-pyrimidinyl)pipérazino)butyl)-3,5-dioxo-(2H, 4H)-1,2,4-triazine 25.

Ce composé est préparé selon le procédé décrit à l'exemple 4 en utilisant au stade d) la 4-(4-méthyl-2-pyrimidinyl)pipérazine.
F = 101°C
CCM : gel de silice 60F254 Merck.
CH₂Cl₂ - MeOH 90-10 Rf= 0,50.

### Exemple 26 : 4-méthyl-2-(4-(4-(4,6-diméthyl-2-pyrimidinyl)pipérazino) butyl)-3,5-dioxo-(2H, 4H)-1,2,4-triazine 26.

Ce composé est préparé selon le procédé décrit à l'exemple 4 en utilisant au stade d) la 4-(4,6-diméthyl-2-pyrimidinyl)pipérazine.
F = 120°C
CCM : gel de silice 60F254 Merck
CH₂Cl₂ - MeOH 90-10 Rf= 0,70.

### Exemple 27 : 4-méthyl-2-(4-(4-(4,6-diméthyl-2-pyrimidinyl)pipérazino) butyl)-3,5-dioxo-(2H, 4H)-1,2,4-triazine 27.

Ce composé est préparé selon le procédé décrit à l'exemple 4 en utilisant au stade d) la 4-(4-trifluorométhyl pyrimidinyl)pipérazine.
F = 95°C
CCM : gel de silice 60F254 Merck
CH₂Cl₂ - MeOH 95-5 Rf= 0,36.

### Exemple 28 : 4-méthyl-2-(4-(4-(4-méthoxy-2-pyrimidinyl)pipérazino) butyl)-3,5-dioxo-(2H, 4H)-1,2,4-triazine 28.

Ce composé est préparé selon le procédé décrit à l'exemple 4 en utilisant au stade d) la 4-(4-méthoxy-2-pyrimidinyl)pipérazine.
F = 87°C
CCM : gel de silice 60F254 Merck
CH₂Cl₂ - MeOH Rf= 0,40.

### Exemple 29 : 4-méthyl-2-(4-(4-(3 -méthoxy-2-pyridyl)pipérazino)butyl)-3,5-dioxo-(2H, 4H)-1,2,4-triazine fumarate 29.

Ce composé est préparé selon le procédé décrit à l'exemple 4 en utilisant au stade d) la (3-méthoxy-2-pyridyl)pipérazine puis en salifiant par l'acide fumarique.
F = 182°C
CCM : gel de silice 60F254 Merck
CH₂Cl₂ - MeOH 90-10 Rf= 0,33.

### Exemple 30 : 4-méthyl-2-(4-(4-(2-pyrimidinyl)pipérazino)butyl)-3,5-dioxo-(2H, 4H)-1,2,4-triazine fumarate 30.

Ce composé est obtenu en salifiant le composé 2 par l'acide fumarique.
F = 134°C
CCM : gel de silice 60F254 Merck
CHCl₃ - MeOH 95-5 Rf= 0,40.

Les composés de l'invention ont été soumis à des essais pharmacologiques qui ont mis en évidence leur intérêt comme substances actives en thérapeutique.

Ainsi, ils ont fait l'objet d'une étude portant sur leur affinité pour les récepteurs sérotoninergiques du type 5-HT_{1A}.

L'étude de la liaison au récepteur 5-HT_{1A} est réalisée comme décrit par Sleight et Peroutka (Naunyn-Schmiedebergs Arch. Pharmacol., 343, 106-116, 1991). Pour ces expérimentations, des cortex cérébraux de rat sont utilisés. Le cerveau est disséqué et le cortex est homogénéisé dans 20 volumes de tampon Tris-HCl (50 mM, pH 7,4 à 25°C) maintenu à 4°C. L'homogénat est centrifugé à 39 000 x g pendant 10 minutes, le culot de centrifugation est mis en suspension dans le même volume de tampon et centrifugé à nouveau. Après une nouvelle mise en suspension dans les mêmes conditions, l'homogénat est incubé pendant 10 minutes à 37°C puis centrifugé à nouveau. Le culot final est mis en suspension dans 80 volumes de tampon de réaction contenant : pargyline (10⁻⁵M), CaCl₂ (4 mM) et acide ascorbique (0,1 %) dans du Tris-HCl (50 mM, pH 7,4 à 25°C). La concentration finale de tissu dans le milieu d'incubation est 10 mg/tube.

Dans les expériences de saturation, les tubes de réaction contiennent 0,1 ml dé différentes concentrations (comprises entre 0,06 et 8 nM) de [³H]8-OH-DPAT , 0,1ml de tampon de réaction ou de 5-HT (10⁻⁵M, pour déterminer la liaison non-spécifique) et 0,8 ml de tissu.

Les expériences de déplacement sont réalisées comme décrit par Sleight et Peroutka (Naunvn-Schmiedebergs Arch. Pharmacol., 343, 106-116, 1991). Toutes les dilutions de produits à étudier sont réalisées dans le tampon de réaction. Les tubes de réaction contiennent 0,1 ml de [³H]8-OH-DPAT (0,2 nM), 0,1 ml de produit à tester 6-7 concentrations (dilutions successives au 1/10) et 0,8 ml de tissu). Si l'affinité présumée des produits se situe dans le domaine nanomolaire, la plus faible concentration testée est 10⁻¹¹ M, si le produit a une affinité présumée faible, la plus forte concentration testée est 10⁻⁴M. Les tubes de réaction sont incubés à 23°C pendant 30 minutes puis rapidement filtrés sous vide sur filtres Whatman GF/B, les tubes sont rinçés avec 2 x 5 ml de tampon Tris-HCl (50 mM, pH 7,4 à 25°C). La radioactivité recueillie sur le filtre est analysée en scintillation liquide en ajoutant 4 ml de liquide scintillant (Emulsifer Safe, Packard). Toutes les expériences sont réalisées en triple et répétées au moins 3 fois.

La constante de dissociation (K_{D}) et le nombre maximum de sites de liaison (Bmax) pour le radioligand sont estimés à partir des expériences de saturation en utilisant le programme de régression non-linéaire EBDA/LIGAND. Cette méthode admet que la valeur du coefficient de Hill n'est pas différente de l'unité.

Les données des expériences de déplacement sont analysées respectivement avec les modèles un site et deux sites et le F calculé permet de déterminer si le modèle deux sites est plus représentatif des données obtenues que le modèle un site. Les valeurs de pKi sont données sous forme de moyenne ± SEM de 3 à 5 expériences.

Le tableau 1 donne, à titre d'exemple, les pKi 5-HT_{1A} pour certains dérivés de l'invention, par rapport à la Buspirone.

**Tableau 1 :**

| affinité pour le récepteur 5-HT_{1A} | |
|---|---|
| Composé n° | pKi |
| 1 | 9.26 |
| 3 | 9.62 |
| 4 | 9.57 |
| 9 | 8.34 |
| 10 | 10.49 |
| 11 | 9.88 |
| 12 | 8.19 |
| 13 | 8.58 |
| 14 | 9.57 |
| 15 | 8.55 |
| 16 | 9.36 |
| 21 | 8.58 |
| 22 | 9.50 |
| 23 | 9.04 |
| 24 | 9.10 |
| 25 | 9.14 |
| 26 | 9.19 |
| 27 | 9.23 |
| 28 | 9.07 |
| 30 | 8.48 |
| Buspirone | 7.65 |

Les résultats des essais montrent que les composés de formule générale I possèdent une haute affinité pour les récepteurs sérotoninergiques de type 5-HT_{1A}.

L'activité centrale des composés de l'invention a été évaluée par leur capacité de provoquer le syndrome 5-HT, qui est caractérisé par une flexion et une extension alternées des pattes avant (reciprocal fore-paw treading : FPT), la rétraction de la lèvre inférieure (lower-lip retraction : LLR) et par une posture où la surface ventrale de l'animal est en contact avec le sol de la cage avec les pattes arrières étendues (flat body posture : FBP).

Les expériences de l'évaluation du syndrome 5-HT sont réalisées chez le rat mâle (Sprague Dawley) selon la méthode décrite par F.C COLPAERT et al (Drug Dev. Res., 26, 21-48 ; 1992).

Le tableau 2 donne, à titre d'exemple, les doses actives (ED₅₀) pour certains dérivés de l'invention par rapport à un produit de référence, la Buspirone.

**Tableau 2 :**

| Syndrome 5-HT | | | |
|---|---|---|---|
| Composé n° | ED₅₀ : mg/kg ip | | |
| | FBP | LLR | FPT |
| 3 | 0.31 | 0.08 | 0.31 |
| 9 | 0.08 | 0.08 | 0.31 |
| 10 | 0.02 | 0.005 | 0.02 |
| Buspirone | 5.0 | 1.25 | > 40 |

Les résultats des essais montrent que les composés de formule générale I possèdent, in vitro, une haute affinité pour les récepteurs sérotoninergiques de type 5-HT_{1A}. In vivo, ils montrent une activité agoniste au niveau de ces récepteurs.

Les composés de l'invention peuvent donc être utiles pour le traitement de l'anxiété, la dépression, la douleur, la schizophrénie, la maladie d'Alzheimer, les troubles de sommeil, pour la régularisation de la prise de nourriture, pour la régularisation de la sécrétion gastrique, et pour le traitement des désordres vasculaires, cardiovasculaires et cérébrovasculaires tels que l'hypertension ou la migraine.

Les composés de la présente invention présentent une activité sur la prolifération des lymphocytes T et peuvent donc être utiles pour le traitement du virus HIV.

Les préparations pharmaceutiques contenant ces principes actifs peuvent être mises en forme pour l'administration par voie orale, rectale ou parentérale, par exemple sous la forme de capsules, comprimés, granulés, gélules, solutés liquides, sirops ou suspensions buvables, et contenir les excipients appropriés.

Il est également possible d'y associer d'autres principes actifs pharmaceutiquement et thérapeutiquement acceptables.

## Revendications

1. Dérivés de 3,5-dioxo-(2H, 4H)-1,2,4-triazine correspondant à la formule I. 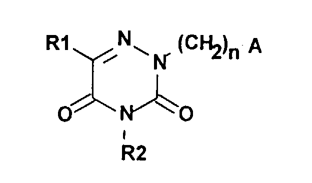 dans laquelle
- R₁ représente l'hydrogène, un radical alcoyle en C₁-C₄, phényl alcoyle en C₁-C₄ ou phényle, le noyau phényle étant éventuellement substitué par un ou plusieurs groupements tels que alcoyle en C₁-C₃, hydroxyle, trifluorométhyle ou halogène,
- R₂ représente l'hydrogène ou un radical alcoyle en C₁-C₄,
- n peut prendre les valeurs entières de 2 à 6,
- A représente un groupement de type
. aryl piperazino II 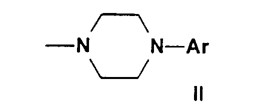 le groupement Ar représentant lui-même une structure aromatique telle que phényle, naphtyle, pyrimidyle, pyridyle, éventuellement substituée par un ou plusieurs groupements tels que alcoyle en C₁-C₃, alcoxy en C₁-C₃, hydroxy, trifluorométhyle ou halogène,
. benzodioxanyl méthylamino ou pyridodioxanyl méthyl amino III 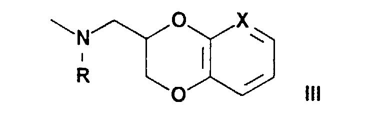 dans lequel R représente l'hydrogène ou un groupement alcoyle en C₁-C₃ et X représente un atome d'azote ou de carbone ainsi que les sels d'addition avec les acides pharmaceutiquement acceptables, et les différents énantiomères dans le cas de composés possédant un carbone asymétrique.

2. Composés de formule générale I selon la revendication 1 caractérisés en ce qu'ils sont choisis parmi :
• 2-(4-(4-(3-trifluorométhylphényl) pipérazino) butyl)-3,5-dioxo-(2H, 4H)-1,2,4-triazine, chlorhydrate,
• 4-méthyl-2-(4-(4-(3-trifluorométhylphényl) piperazino) butyl)-3,5-dioxo-(2H, 4H)-1,2,4-triazine,
• 4-méthyl-2-(4-(4-(3-trifluorométhylphényl) pipérazino) butyl)-3,5-dioxo-(2H, 4H)-1,2,4-triazine chlorhydrate,
• 4-méthyl-2-(4-(4-(3-chlorophényl) pipérazino) butyl)-3,5-dioxo-(2H, 4H)-1,2,4-triazine,
• 6-phényl-2-(4-(4-(3-trifluorométhylphényl) pipérazino) butyl)-3,5-dioxo-(2H, 4H)-1,2,4-triazine chlorhydrate,
• 4-méthyl-6-phényl-2-(4-(4-(3-trifluorométhylphényl) pipérazino) butyl)-3,5-dioxo-(2H, 4H)-1,2,4-triazine chlorhydrate,
• 2-(4-(4-(2-pyrimidinyl) pipérazino) butyl)-3,5-dioxo-(2H, 4H)-1,2,4-triazine,
• 4-méthyl-2-(4-(4-(2-pyrimidinyl) pipérazino) butyl)-3,5-dioxo-(2H, 4H)-1,2,4-triazine,
• 4-méthyl-2-(4-(4-(7-méthoxy-1-naphtyl) pipérazino) butyl)-3,5-dioxo-(2H, 4H)-1,2,4-triazine fumarate,
• 4-méthyl-2-(4-(4-(2-méthoxyphényl) pipérazino) butyl)-3,5-dioxo-(2H, 4H)-1,2,4-triazine,
• 6-méthyl-2-(4-(4-(3-trifluorométhylphényl) pipérazino) butyl)-3,5-dioxo-(2H, 4H)-1,2,4-triazine,
• 4,6-diméthyl-2-(4-(4-(3-trifluorométhylphényl) pipérazino) butyl)-3,5-dioxo-(2H, 4H)-1,2,4-triazine chlorhydrate,
• 4-méthyl-2-(4-(1,4-benzodioxan-2-yl méthylamino) butyl)-3,5-dioxo-(2H, 4H)-1,2,4-triazine,
• 4-méthyl-2-(4-(N-méthyl N-1,4-benzodioxan-2-yl méthylamino) butyl)-3,5-dioxo-(2H, 4H)-1,2,4-triazine fumarate,
• 4-méthyl-2-(4-(2,3-dihydro-1,4-dioxino-[2,3-b]-pyridin-2-yl méthylamino) butyl-3,5-dioxo-(2H, 4H)-1,2,4-triazine fumarate,
• 2-(3-(4-(3-trifluorométhylphényl) pipérazino) propyl)-3,5-dioxo-(2H, 4H)-1,2,4-triazine,
• 2-(2-(4-(3-trifluorométhylphényl) pipérazino) éthyl)-3,5-dioxo-(2H,4H)-1,2,4-triazine chlorhydrate,
• 4-méthyl-2-(3-(1,4-benzodioxan-2-yl méthylamino) propyl)-3,5-dioxo-(2H, 4H)-1,2,4-triazine chlorhydrate,
• 4-méthyl-2-(3-(2,3-dihydro-1,4-dioxino-[2,3-b]-pyridin-2-yl méthylamino) propyl)-3,5-dioxo-(2H, 4H)-1,2,4-triazine fumarate,
• 4-méthyl-2-(4-(4-(3,4-dichlorophényl) pipérazino) butyl)-3,5-dioxo-(2H, 4H)-1,2,4-triazine,
• 4-méthyl-2-(4-(4-(2,3-dichlorophényl) pipérazino) butyl)-3,5-dioxo-(2H, 4H)-1,2,4-triazine,
• 4-méthyl-2-(4-(4-(3,5-dichlorophényl) pipérazino) butyl)-3,5-dioxo-(2H, 4H)-1,2,4-triazine,
• 4-méthyl-2-(4-(4-(4-chloro-2-pyrimidinyl) pipérazino) butyl-3,5-dioxo-(2H, 4H)-1,2,4-triazine,
• 4-méthyl-2-(4-(4-(4-methyl-2-pyrimidinyl) pipérazino) butyl)-3,5-dioxo-(2H, 4H)-1,2,4-triazine,
• 4-méthyl-2-(4-(4-(4,6-diméthyl-2-pyrimidinyl) pipérazino) butyl)-3,5-dioxo-(2H, 4H)-1,2,4-triazine,
• 4-méthyl-2-(4-(4-(4-trifluorométhyl-2-pyrimidinyl) pipérazino) butyl)-3,5-dioxo-(2H, 4H)-1,2,4-triazine,
• 4-méthyl-2-(4-(4-(4-méthoxy-2-pyrimidinyl) pipérazino) butyl)-3,5-dioxo-(2H, 4H)-1,2,4-triazine,
• 4-méthyl-2-(4-(4-(3-méthoxy-2-pyridyl) pipérazino) butyl)-3,5-dioxo-(2H, 4H)-1,2,4-triazine fumarate,
• 4-méthyl-2-(4-(4-(2-pyrimidinyl) pipérazino) butyl)-3,5-dioxo-(2H, 4H)-1,2,4-triazine fumarate.

3. Procédé de préparation des composés chimiques selon les revendications 1 et 2, caractérisé :
a) en ce que l'on traite un composé de formule générale IV : 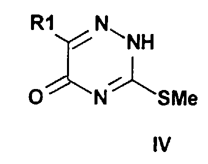 dans laquelle R₁ est tel que défini dans la revendication 1 avec un dérivé dihalogéné V,
Hal-(CH₂)n-Hal' V
dans laquelle n est tel que défini dans la revendication 1, et Hal et Hal' représentent un halogène de préférence respectivement le chlore pour Hal et le brome pour Hal',
b) en ce que, après hydrolyse acide, on condense un dérivé de formule VI ou VII : 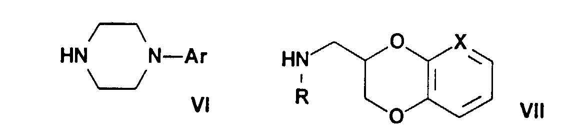 dans lesquels Ar, X et R sont tels que définis dans la revendication 1,
c) en ce que l'on alcoyle (R₂ # H) par un dérivé R₂Y dans lequel R₂ est tel que défini dans la revendication 1 et Y représente le chlore, le brome ou l'iode.

4. Procédé de préparation de composés chimiques selon les revendications 1, 2 et 3, caractérisé :
a) en ce que l'on traite un composé de formule VIII : 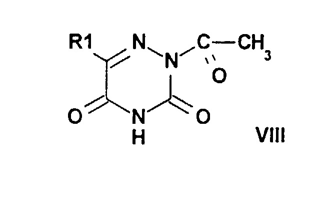 dans lequel R₁ est tel que défini dans la revendication 1 par un halogénure d'alcoyle R₂Y dans lequel R₂ et Y sont tels que définis aux revendications 1 et 3,
b) en ce que l'on désacétyle un milieu acide puis condense un composé dihalogéné V, puis un dérivé VI ou VII, les composés V, VI et VII étant tels que définis à la revendication 3.

5. A titre de médicaments, les composés définis selon l'une des revendications 1 ou 2.

6. Médicaments selon la revendication 5, utilisables dans le traitement des maladies nécessitant des agonistes de récepteurs 5HT_{1A} ou pour le traitement de l'anxiété, la dépression, la douleur, la schizophrénie, la maladie d'Alzheimer, les troubles du sommeil, la régularisation de prise de nourriture, la régularisation de la sécrétion gastrique, le traitement des désordres vasculaires, cardiovasculaires et cérébrovasculaires, ou du virus HIV.

7. Composition pharmaceutique caractérisée en ce qu'elle contient un composé défini selon l'une des revendications 1 et 2.

8. Composition pharmaceutique caractérisée en ce qu'elle contient un composé défini selon l'une des revendications 1 et 2 en association avec tout excipient approprié.

9. Composition pharmaceutique caractérisée en ce qu'elle contient un composé défini selon l'une des revendications 1 et 2 associé à un autre principe actif.

## Patentansprüche

1. 3,5-Dioxo-(2H,4H)-1,2,4-triazin-Derivat der Formel I 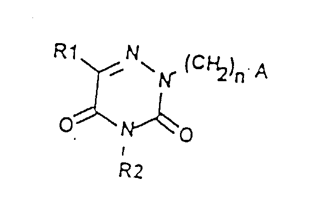 wobei
- R₁ Wasserstoff, ein C₁-C₄-Alkyl-, C₁-C₄-Alkylphenyl- oder Phenylradikal darstellt, wobei der Phenylkern gegebenenfalls durch eine oder mehrere Gruppen wie C₁-C₃-Alkyl, Hydroxyl, Trifluormethyl oder Halogen substituiert sein kann,
- R₂ Wasserstoff oder ein C₁-C₄-Alkylradikal darstellt,
- n Werte zwischen 2 und 6 annehmen kann,
- A eine Gruppe darstellt vom Typ
• Arylpiperazino, II 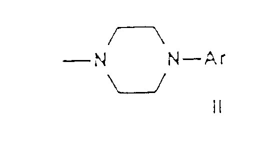 wobei die Gruppe Ar selbst eine aromatische Struktur darstellt wie Phenyl, Naphthyl, Pyrimidyl, Pyridyl, gegebenenfalls substituiert durch eine oder mehrere Gruppen wie C₁-C₃-Alkyl, C₁-C₃-Alkoxy, Hydroxy, Trifluormethyl oder Halogen,
• Benzodioxanylmethylamino oder Pyridodioxanylmethylamino III 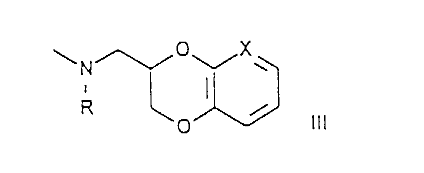 wobei R Wasserstoff oder eine C₁-C₃-Alkylgruppe darstellt und X ein Stickstoff- oder Kohlenstoffatom darstellt,
sowie ihre Additionssalze mit pharmazeutisch verträglichen Säuren, und die verschiedenen Enantiomere im Fall von Verbindungen mit einem asymmetrischen Kohlenstoffatom.

2. Verbindungen der allgemeinen Formel I nach Anspruch 1, dadurch charakterisiert, daß sie ausgewält werden aus:
• 2-(4-(4-(3-Trifluoromethylphenyl)-piperazino)-butyl)-3,5-dioxo-(2H,4H)-1,2,4-triazin-Hydrochlorid,
• 4-Methyl-2-(4-(4-(3-trifluoromethylphenyl)-piperazino)-bulyl)-3,1-dioxo-(2H,4H)-1,2,4-triazin,
• 4-Methyl-2-(4-(4-(3-trifluoromethylphenyl)-piperazino)-butyl)-3,5-dioxo-(2H,4H)-1,2,4-triazin-Hydrochlorid,
• 4-Methyl-2-(4-(4-(3-chlorophenyl)-piperazino)-butyl)-3,5-dioxo-(2H,4H)-1,2,4-triazin,
• 6-Phenyl-2-(4-(4-(3-tritluoromethylphenyl)-piperazino)-butyl)-3,5-dioxo-(2H,4H)-1,2,4-triazin-Hydrochlorid,
• 4-Methyl-6-Phenyl-2-(4-(4-(3-trifluoromethylphenyl)-piperazino)-butyl)-3,5-dioxo-(2H,4H)-1,2,4-triazin-Hydrochlorid,
• 2-(4-(4-(2-Pyrimidinyl)-piperazino)-butyl)-3,5-dioxo-(2H,4H)-1,2,4-triazin,
• 4-Methyl-2-(4-(4-(3-pyrimidinyl)-piperazino)-butyl)-3,5-dioxo-(2H,4H)-1,2,4-triazin,
• 4-Methyl-2-(4-(4-(7-methoxy-1-naphthyl)-piperazino)-butyl)-3,5-dioxo-(2H,4H)-1,2,4-triazin-Fumarat,
• 4-Methyl-2-(4-(4-(2-methoxyphenyl)-piperazino)-butyl)-3,5-dioxo-(2H,4H)-1,2,4-triazin,
• 6-Methyl-2-(4-(4-(3-trifluoromethylphenyl)-piperazino)-butyl)-3,5-dioxo-(2H,4H)-1,2,4-triazin,
• 4, 6-Dimethyl-2-(4-(4-(3-trifluoromethylphenyl)-piperazino)-butyl)-3,5-dioxo-(2H ,4H)- 1,2,4-triazin-Hydrochlorid,
• 4-Methyl-2-(4-(1,4-benzodioxan-2-yl-methylamino)-butyl)-3,5-dioxo-(2H,4H)-1,2,4-triazin,
• 4-Methyl-2-(4-(N-methyl-N-1,4-benzodioxan-2-yl-methylamino)-bulyl)-3,5-dioxo-(2H,4H)-1,2,4-triazin-Fumarat,
• 4-Methyl-2-(4-(2,3-dihydro-1,4-dioxino-[2,3-b]pyridin-2-yl-methylamino)-butyl)-3,5-dioxo-(2H,4H)-1,2,4-triazin-Fumarat,
• 2-(3-(4-(3-Trifluoromethylphenyl)piperazino)propyl)-3,5-dioxo-(2H,4H)-1,2,4-triazin,
• 2-(2-(4-(3-Trifluoromethylphenyl)piperazino)ethyl)-3,5-dioxo-(2H,4H)-1,2,4-triazin-Hydrochlorid,
• 4-Methyl-2-(3-(1,4-benzodioxan-2-yl-methylamino)-propyl)-3,5-dioxo-(2H,4H)-1,2,4-triazin-Hydrochlorid,
• 4-Methyl-2 -(3-(2,3-dihydro-1,4-dioxino-[2,3-b]-pyridin-2-yl-methylamino)-propyl)-3,5-dioxo-(2H,4H)-1,2,4-triazin-Fumarat,
• 4-Methyl-(2-(4-(4-(3,4-dichlorophenyl)-piperazino)-butyl)-3,1-dioxo-(2H,4H)-1,2,4-triazin,
• 4-Methyl-(2-(4-(4-(2,3-dichlorophenyl)-piperazino)-butyl)-3,5-dioxo-(2H,4H)-1,2,4-triazin,
• 4-Methyl-(2-(4-(4-(3,5-dichlorophenyl)-piperazino)-butyl)-3,2-dioxo-(2H,4H)-1,2,4-triazin,
• 4-Methyl-(2-(4-(4-(4-chloro-2-pyrimidinyl)-piperazino)-butyl)-3,5-dioxo-(2H,4H)-1,2,4-triazin,
• 4-Methyl-(2-(4-(4-(4-methyl-2-pyrimidinyl)-piperazino)-butyl)-3,5-dioxo-(2H,4H)-1,2,4-triazin,
• 4-Methyl-(2-(4-(4-(4,6-dimethyl-2-pyrimidinyl)-piperazino)-butyl)-3,5-dioxo-(2H,4H)-1,2,4-triazin,
• 4-Methyl-(2-(4-(4-(4-trifluoromethyl-2-pyrimidinyl)-piperazino)-butyl)-3,5-dioxo-(2H,4H)-1,2,4-triazin,
• 4-Methyl-(2-(4-(4-(4-methoxy-2-pyrimidinyl)-piperazino)-butyl)-3,5-dioxo-(2H,4H)-1,2,4-triazin,
• 4-Methyl-(2-(4-(4-(3-methoxy-2-pyridyl)-piperazino)-butyl)-3,5-dioxo-(2H,4H)-1,2,4-triazin-Fumarat,
• 4-Methyl-(2-(4-(4-(2-pyrimidinyl)-piperazino)-butyl)-3,5-dioxo-(2H,4H)-1,2,4-triazin-Fumarat.

3. Verfahren zur Herstellung von chemischen Verbindungen nach den Ansprüchen 1 und 2, dadurch charakterisiert, daß:
a) man eine Verbindung der allgemeinen Formel IV: 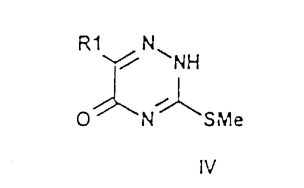 wobei R₁ wie in Anspruch 1 definiert ist, mit einem Dihalogenderivat V behandelt
Hal-(CH₂)n-Hal' V
wobei n wie in Anspruch 1 definiert ist, und Hal und Hal' ein Halogen darstellen, bevorzugt jeweils Chlor für Hal und Brom für Hal' steht,
b) man nach saurer Hydrolyse ein Derivat der Formel VI oder VII 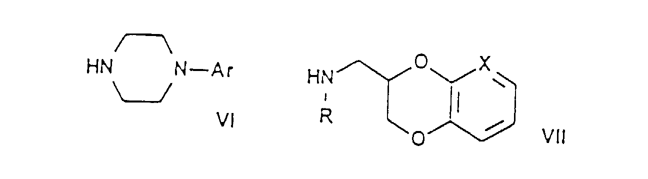 wobei Ar, X und R wie in Anspruch 1 definiert sind, kondensiert
c) man mit einem Derivat R₂Y alkyliert (R₂ # H), wobei R₂ definiert ist wie in Anspruch 1 und Y Chlor, Brom oder Iod darstellt.

4. Verfahren zur Herstellung von chemischen Verbindungen nach den Ansprüchen 1, 2 und 3, dadurch charakterisiert, daß:
a) man eine Verbindung der Formel VIII: 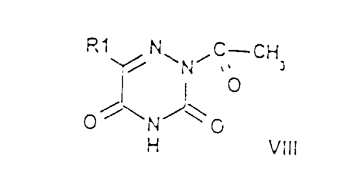 wobei R₁ wie in Anspruch 1 definiert ist, mit einem Alkylhalogenid R₂Y behandelt, wobei R₉ und Y wie in Ansprüchen 1 und 3 definiert sind,
b) man in saurem Milieu deacetyliert und anschließend eine Dihalogenverbindung V, anschließend ein Derivat VI oder VII kondensiert, wobei die Verbindungen V, VI und VII wie in Anspruch 3 definiert sind.

5. Verbindungen nach einem der Ansprüche 1 oder 2 als Medikamente.

6. Medikamente nach Anspruch 5, einsetzbar in der Behandlung von 5HT_{1A}-Rezeptor-Agonisten erfordernden Erkrankungen oder für die Behandlung von Angstzuständen, Depressionen, Schmerz, Schizophrenie, Alzheimer-Krankheit, Schlafstörungen, Regelung der Nahrungsaufnahme, Regelung der gastrischen Sekretion, Behandlung von vaskulären, kardiovaskulären und cerebrovaskulären Erkrankungen, oder des HIV-Virus.

7. Pharmazeutische Zusammensetzung, dadurch charakterisiert, daß sie eine in einen der Ansprüche 1 und 2 definierte Verbindung enthält.

8. Pharmazeutische Zusammensetzung, dadurch charakterisiert, daß sie eine in einen der Ansprüche 1 und 2 definierte Verbindung mit einem geeigneten Excipienten enthält.

9. Pharmazeutische Zusammensetzung, dadurch charakterisiert, daß sie eine in einen der Ansprüche 1 und 2 definierte Verbindung in Verbindung mit einem weiteren aktiven Wirkstoff enthält.

## Claims

1. 3,5-Dioxo-(2H,4H)-1,2,4-triazine derivatives corresponding to the formula I. 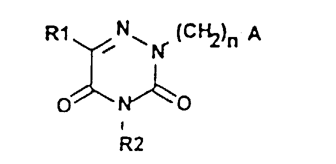 in which
- R₁ represents hydrogen, a (C₁-C₄) alkyl, phenyl(C₁-C₄ alkyl) or phenyl radical, the phenyl ring being optionally substituted by one or more groups such as (C₁-C₃) alkyl, hydroxyl, trifluoromethyl or halogen,
- R₂ represents hydrogen or a (C₁-C₄) alkyl radical,
- n can be an integer from 2 to 6,
- A represents a group of the type
. arylpiperazino II 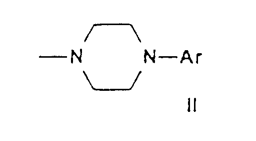 the Ar group itself representing an aromatic structure such as phenyl, naphthyl, pyrimidyl, pyridyl, optionally substituted by one or more groups such as (C₁-C₃) alkyl, (C₁-C₃) alkoxy, hydroxyl, trifluoromethyl or halogen,
. benzodioxanylmethylamino or pyridodioxanylmethylamino III 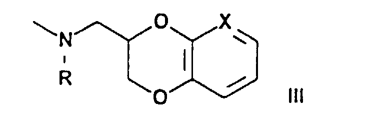 in which R represents hydrogen or a (C₁-C₃) alkyl group and X represents a nitrogen or carbon atom as well as the addition salts with pharmaceutically acceptable acids, and the various enantiomers in the case of compounds having an asymmetric carbon.

2. Compounds of general formula I according to Claim 1, characterized in that they are chosen from:
· 2-(4-(4-(3-trifluoromethylphenyl)piperazino)butyl)-3,5-dioxo- (2H,4H) -1,2,4-triazine, hydrochloride,
· 4-methyl-2-(4-(4-(3-trifluoromethylphenyl)piperazino)butyl)-3,5-dioxo-(2H,4H)-1,2,4-triazine,
· 4-methyl-2-(4-(4-(3-trifluoromethylphenyl)piperazino)butyl)-3,5-dioxo-(2H,4H)-1,2,4-triazine hydrochloride,
· 4-methyl-2- (4- (4- (3-chlorophenyl)piperazino)butyl)-3,5-dioxo-(2H,4H)-1,2,4-triazine,
· 6-phenyl-2-(4-(4-(3-trifluoromethyl-phenyl)piperazino)butyl)-3,5-dioxo-(2H,4H)-1,2,4-triazine hydrochloride,
· 4-methyl-6-phenyl-2-(4-(4-(3-trifluoromethyl-phenyl)piperazino)butyl)-3,5-dioxo-(2H,4H)-1,2,4-triazine hydrochloride,
· 2-(4-(4-(2-pyrimidinyl)piperazino)butyl)-3,5-dioxo-(2H,4H)-1,2,4-triazine,
· 4-methyl-2-(4-(4-(2-pyrimidinyl)piperazino)butyl)-3,5-dioxo-(2H,4H)-1,2,4-triazine,
· 4-methyl-2-(4-(4-(7-methoxy-1-naphthyl)piperazino)butyl)-3,5-dioxo-(2H,4H)-1,2,4-triazine fumarate,
· 4-methyl-2-(4-(4-(2-methoxyphenyl)piperazino)butyl)-3,5-dioxo-(2H,4H)-1,2,4-triazine,
· 6-methyl-2-(4-(4-(3-trifluoromethylphenyl)piperazino)butyl)-3,5-dioxo-(2H,4H)-1,2,4-triazine,
· 4,6-dimethyl-2-(4-(4-(3-trifluoromethyl-phenyl)piperazino)butyl)-3,5-dioxo-(2H,4H)-1,2,4-triazine hydrochloride,
· 4-methyl-2-(4-(1,4-benzodioxan-2-ylmethylamino)butyl)-3,5-dioxo-(2H,4H)-1,2,4-triazine,
· 4-methyl-2-(4-(N-methyl-N-1,4-benzodioxan-2-yl-methylamino)butyl)-3,5-dioxo-(2H,4H)-1,2,4-triazine fumarate,
· 4-methyl-2-(4-(2,3-dihydro-1,4-dioxino-[2,3-b]-pyridin-2-ylmethylamino)butyl-3,5-dioxo-(2H,4H)-1,2,4-triazine fumarate,
· 2- (3- (4- (3-trifluoromethylphenyl)piperazino)propyl)-3,5-dioxo-(2H,4H)-1,2,4-triazine,
· 2-(2-(4-(3-trifluoromethylphenyl)piperazino)ethyl)-3,5-dioxo-(2H,4H)-1,2,4-triazine hydrochloride,
· 4-methyl-2-(3-(1,4-benzodioxan-2-ylmethyl-amino)propyl)-3,5-dioxo-(2H,4H)-1,2,4-triazine hydrochloride,
· 4-methyl-2-(3-(2,3-dihydro-1,4-dioxino-[2,3-b]-pyridin-2-ylmethylamino)propyl)-3,5-dioxo- (2H,4H)-1,2,4-triazine fumarate,
· 4-methyl-2-(4-(4-(3,4-dichlorophenyl)piperazino)butyl)-3,5-dioxo-(2H,4H)-1,2,4-triazine,
· 4-methyl-2-(4-(4-(2,3-dichloro-phenyl)piperazino)butyl)-3,5-dioxo-(2H,4H)-1,2,4-triazine,
· 4-methyl-2-(4-(4-(3,5-dichloro-phenyl)piperazino)butyl)-3,5-dioxo-(2H,4H)-1,2,4-triazine,
· 4-methyl-2-(4-(4-(4-chloro-2-pyrimidinyl)piperazino)butyl)-3, 5-dioxo-(2H,4H)-1,2,4-triazine,
· 4-methyl-2-(4-(4-(4-methyl-2-pyrimidinyl)piperazino)butyl)-3,5-dioxo-(2H,4H)-1,2,4-triazine,
· 4-methyl-2-(4-(4-(4,6-dimethyl-2-pyrimidinyl)piperazino)butyl)-3, 5-dioxo-(2H,4H)-1,2,4-triazine,
· 4-methyl-2-(4-(4-(4-trifluoromethyl-2-pyrimidinyl)piperazino)butyl)-3,5-dioxo-(2H, 4H)-1,2,4-triazine,
· 4-methyl-2-(4-(4-(4-methoxy-2-pyrimidinyl)piperazino)butyl)-3, 5-dioxo- (2H,4H)-1,2,4-triazine,
· 4-methyl-2-(4-(4-(3-methoxy-2-pyridyl)piperazino)butyl)-3,5-dioxo-(2H,4H)-1,2,4-triazine fumarate,
· 4-methyl-2-(4-(4-(2-pyrimidinyl)piperazino)butyl)-3,5-dioxo-(2H,4H)-1,2,4-triazine fumarate.

3. Process for the preparation of the chemical compounds according to Claims 1 and 2, characterized:
a) in that a compound of general formula IV: 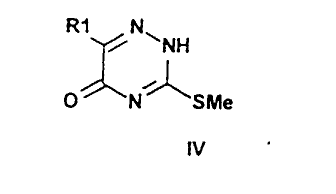 in which R₁ is as defined in Claim 1, is treated with a dihalogenated derivative V,
Hal-(CH₂)n-Hal' V
in which n is as defined in Claim 1, and Hal and Hal' represent a halogen, preferably respectively chlorine for Hal and bromine for Hal',
b) in that, after acid hydrolysis, a derivative of formula VI or VII: 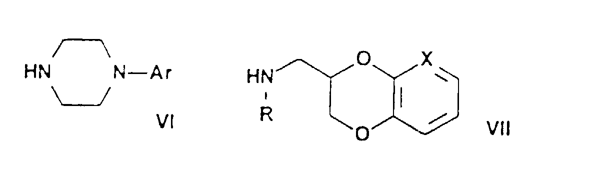 in which Ar, X and R are as defined in Claim 1, is condensed,
c) in that alkylation is performed (R₂#H) with a derivative R₂Y in which R₂ is as defined in Claim 1 and Y represents chlorine, bromine or iodine.

4. Process for the preparation of chemical compounds according to Claims 1, 2 and 3, characterized:
a) in that a compound of formula VIII: 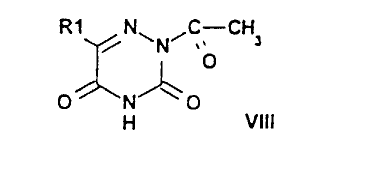 in which R₁ is as defined in Claim 1, is treated with an alkyl halide R₂Y in which R₂ and Y are as defined in Claims 1 and 3,
b) in that deacetylation is carried out in an acidic medium, then a dihalogenated compound V, then a derivative VI or VII are condensed, the compounds V, VI and VII being as defined in Claim 3.

5. As medicaments, the compounds defined according to either of Claims 1 and 2.

6. Medicaments according to Claim 5, which can be used in the treatment of diseases requiring agonists of 5HT_{1A} receptors or for the treatment of anxiety, depression, pain, schizophrenia, Alzheimer's disease, sleep disorders, regulation of food intake, regulation of gastric secretion, treatment of vascular, cardiovascular and cerebrovascular disorders, or the HIV virus.

7. Pharmaceutical composition characterized in that it contains a compound defined according to either of Claims 1 and 2.

8. Pharmaceutical composition characterized in that it contains a compound defined according to either of Claims 1 and 2 in combination with any appropriate excipient.

9. Pharmaceutical composition characterized in that it contains a compound defined according to either of Claims 1 and 2 combined with another active ingredient.
